# EUROPEAN PATENT APPLICATION

(11) **EP 4 782 548 A1**
(43) Date of publication of application: **29.07.2026**
(21) Application number: 24868312.0
(22) Date of filing: 19.09.2024
(51) Int. Cl.: C12P 21/02, A23L 33/18, A23L 33/19, A61K 38/10, A61P 25/04, A61P 25/22, C07K 7/08, C12N 9/62

(54) **METHOD FOR PRODUCING OPIOID PEPTIDE, METHOD FOR IMPROVING PRODUCTION EFFICIENCY OF OPIOID PEPTIDE, AND ALKALINE PROTEASE COMPOSITION FOR PRODUCING OPIOID PEPTIDE**

(30) Priority: 22.09.2023 JP 2023158444
(71) Applicant: JFR Co., Ltd., Tokyo 177-0044 (JP)
(72) Inventor: YAMAMOTO, Naoyuki, Tokyo 152-8550 (JP); CUI, Yingjie, Tokyo 152-8550 (JP)
(74) Representative: Papula Oy
(86) International application number: PCT/JP2024/033457
(87) International publication number: WO 2025/063235

(57) **Abstract**

The problem addressed by the present invention is to provide an efficient method for producing an opioid peptide. The problem can be solved by a method for producing an opioid peptide, the method comprising a step for bringing a koji mold alkaline protease of the present invention that recognizes the amino acid sequence of SLXX and cleaves between the two Xs into contact with A2-type casein to produce a peptide comprising the amino acid sequence represented by SEQ ID NO: 2.

## Description

### TECHNICAL FIELD

The present invention relates to a method for producing an opioid peptide, a method for improving production efficiency of an opioid peptide, and an alkaline protease composition for producing an opioid peptide. According to the present invention, an opioid peptide can be efficiently produced.

### BACKGROUND ART

In recent years, various stress-induced diseases have been reported, and opioid receptor agonists have been developed as analgesic drugs or neurotropic drugs with the expectation of a morphine-like activity. On the other hand, peptides that are obtained by enzymatically degrading food proteins have been reported to have effects on opioid receptors (Non-Patent Document 1) and are expected to be used as food additives that can reduce stress.

### Citation List

### Patent Documents

Patent Document 1: Japanese Unexamined Patent Application, Publication No. 2022-135015

### Non-Patent Documents

Non-Patent Document 1: "Peptides", 1999 (Netherlands), Vol. 20, pp. 957-962.

### DISCLOSURE OF THE INVENTION

### Problems to be Solved by the Invention

The present inventors have found that casein can be degraded by a protease derived from koji mold to obtain an opioid peptide with high opioid receptor agonistic activity (Patent Document 1). However, it was not easy to efficiently obtain the opioid peptide of Patent Document 1. Thus, an object of the present invention is to provide a method for efficiently producing an opioid peptide.

### Means for Solving the Problems

As a result of extensive studies on a method for efficiently producing an opioid peptide, the present inventors have surprisingly found that a new opioid peptide can be efficiently obtained by using a koji mold alkaline protease and A2-type casein. The present invention is based on such a finding. Thus, the present invention relates to:
[1] A method for producing an opioid peptide, the method including bringing a koji mold alkaline protease that recognizes an amino acid sequence SLXX and cleaves between the two Xs into contact with A2-type casein, thereby producing a peptide including an amino acid sequence set forth in SEQ ID NO: 2;
[2] The method for producing an opioid peptide according to [1], in which the koji mold alkaline protease is (a) a koji mold alkaline protease having an amino acid sequence set forth in SEQ ID NO: 1, or (b) a koji mold alkaline protease including an amino acid sequence having 90% or more identity to the amino acid sequence set forth in SEQ ID NO: 1 and capable of producing from the A2-type casein an opioid peptide including the amino acid sequence set forth in SEQ ID NO: 2;
[3] A method for improving production efficiency of an opioid peptide, including bringing a koji mold alkaline protease that recognizes an amino acid sequence SLXX and cleaves between the two Xs into contact with A2-type casein, thereby producing an opioid peptide including an amino acid sequence set forth in SEQ ID NO: 2, the koji mold alkaline protease being recovered as a low-adsorption fraction on an anion exchange resin or as an adsorption fraction on a cation exchange resin in order to separate the koji mold alkaline protease from a contaminant protease;
[4] The method for improving production efficiency of an opioid peptide according to [3], in which the koji mold alkaline protease is (a) a koji mold alkaline protease having an amino acid sequence set forth in SEQ ID NO: 1, or (b) a koji mold alkaline protease including an amino acid sequence having 90% or more identity to the amino acid sequence set forth in SEQ ID NO: 1 and capable of producing from the A2-type casein an opioid peptide including the amino acid sequence set forth in SEQ ID NO: 2;
[5] An alkaline protease composition for producing an opioid peptide including an amino acid sequence set forth in SEQ ID NO: 2 from A2-type casein, the composition including a koji mold alkaline protease that recognizes an amino acid sequence SLXX and cleaves between the two Xs;
[6] The alkaline protease composition according to [5], in which the koji mold alkaline protease is (a) a koji mold alkaline protease having an amino acid sequence set forth in SEQ ID NO: 1, or (b) a koji mold alkaline protease including an amino acid sequence having 90% or more identity to the amino acid sequence set forth in SEQ ID NO: 1 and capable of producing from the A2-type casein an opioid peptide including the amino acid sequence set forth in SEQ ID NO: 2;
[7] An opioid peptide having an amino acid sequence set forth in SEQ ID NO: 2;
[8] An opioid receptor agonistic agent including as an active ingredient the opioid peptide according to [7];
[9] A food composition for agonizing an opioid receptor, the composition including as an active ingredient the opioid peptide according to [7];
[10] An anti-anxiety food composition including as an active ingredient the opioid peptide according to [7];
[11] A pharmaceutical composition for agonizing an opioid receptor, the composition including as an active ingredient the opioid peptide according to [7]; and
[12] An anti-anxiety pharmaceutical composition including as an active ingredient the opioid peptide according to [7].

### Effects of the Invention

A method for producing an opioid peptide of the present invention allows for efficient production of an opioid peptide that exhibits an excellent effect.

### BRIEF DESCRIPTION OF THE DRAWINGS

[FIG. 1] A photograph (A) of fractions of 10, 50, 100, 250, and 500 mM NaCl from a crudely purified enzyme derived from Aspergillus oryzae in DEAE-Sepharose analyzed by SDS-PAGE, and a graph (B) examining an ability of the fractions to produce an opioid peptide.
[FIG. 2] A photograph (A) of A1-type milk or A2-type milk degraded with a purified alkaline protease and analyzed by SDS-PAGE and charts (B) showing the resulting CM-12 peptide.
[FIG. 3] A graph examining production of a CM-12 peptide from A2-type casein using a purified alkaline protease and a crudely purified enzyme.
[FIG. 4] Graphs examining an optimum pH (A) and an optimum temperature (B) of a purified alkaline protease using CM-30.
[FIG. 5] A figure showing cleavage sites of an alkaline protease of the present invention in A2-type casein.

### PREFERRED MODE FOR CARRYING OUT THE INVENTION

### [1] Method for producing opioid peptide

A method for producing an opioid peptide of the present invention includes bringing a koji mold alkaline protease that recognizes an amino acid sequence SLXX and cleaves between the two Xs into contact with A2-type casein, thereby producing a peptide including an amino acid sequence set forth in SEQ ID NO: 2. In the amino acid sequence SLXX, S is serine, L is leucine, and X means any amino acid (any of 20 types of amino acids). The method of producing an opioid peptide of the present invention may include purifying the opioid peptide produced. The opioid peptide can be purified, for example, by adsorption with a hydrophobic resin and elution with a solvent such as ethanol or acetonitrile. To enrich the peptide to a higher purity, the peptide can be eluted with acetonitrile by HPLC. Furthermore, an insoluble peptide can be removed by adsorption through activated carbon.

### <<Alkaline protease>>

An alkaline protease to be used in the present invention is an alkaline protease that is derived from koji mold and that recognizes an amino acid sequence SLXX and cleaves between the two Xs. The alkaline protease is not limited as long as it recognizes the amino acid sequence SLXX and cleaves between the two Xs. For example, it may be (a) a koji mold alkaline protease having an amino acid sequence set forth in SEQ ID NO: 1. It may also be a koji mold alkaline protease consisting of the amino acid sequence set forth in SEQ ID NO: 1. Furthermore, it may also be (b) an alkaline protease including an amino acid sequence having 90% or more identity to the amino acid sequence set forth in SEQ ID NO: 1 and capable of producing from A2-type casein an opioid peptide including an amino acid sequence set forth in SEQ ID NO: 2. The alkaline protease of (b) is not limited as long as it has a function equivalent to the protease of (a) (e.g., 50% or more activity, e.g., 60% or more activity, e.g., 70% or more activity, e.g., 80% or more activity, e.g., 90% or more activity, e.g., equivalent activity to the alkaline protease of (a)). The identity to the amino acid sequence set forth in SEQ ID NO: 1 is preferably 92% or more, more preferably 94% or more, more preferably 96% or more, more preferably 98% or more, and most preferably 99% or more. The amino acid sequence set forth in SEQ ID NO: 1 is as follows:

MQSIKRTLLLLGAILPAVLGAPVQETRRAAEKLPGKYIVTFKPGIDEAKIQEHTTWATNIHQ

RSLERRGATGGDLPVGIERNYKINKFAAYAGSFDDATIEEIRKNEDVAYVEEDQIYYLDGLT

TQKSAPWGLGSISHKGQQSTDYIYDTSAGEGTYAYVVDSGVNVDHEEFEGRASKAYNAAGGQ

HVDSIGHGTHVSGTIAGKTYGIAKKASILSVKVFQGESSSTSVILDGFNWAANDIVSKKRTS

KAAINMSLGGGYSKAFNDAVENAFEQGVLSVVAAGNENSDAGQTSPASAPDAITVAAIQKSN

NRASFSNFGKVVDVFAPGQDILSAWIGSSSATNTISGTSMATPHIVGLSLYLAALENLDGPA

AVTKRIKELATKDVVKDVKGSPNLLAYNGNA

The amino acid sequence having 90% or more identity may be, for example, an amino acid sequence in which one or several amino acids are deleted from, substituted for, inserted into, and/or added to an original amino acid sequence. A polypeptide of the alkaline protease may be a polypeptide including an amino acid sequence in which one or several amino acids are deleted from, substituted for, inserted into, and/or added to the amino acid sequence set forth in SEQ ID NO: 1, as long as it has 90% or more identity to the amino acid sequence set forth in SEQ ID NO: 1. As used herein, the phrase "amino acid sequence in which one or several amino acids are deleted from, substituted for, inserted into, and/or added to" means that the amino acid sequence has been modified by amino acid substitution, etc., or that the amino acid sequence is from an alkaline protease of a closely related microorganism. A number of amino acids to be modified is not particularly limited as long as it has 90% or more identity to the original amino acid sequence and is, for example, 1 to 40, 1 to 30, 1 to 20, 1 to 15, 1 to 10, 1 to 5, 1 to 4, 1 to 3, or 1 to 2. A preferred example of a modified amino acid sequence of an alkaline protease that can be used in the present invention can be an amino acid sequence with one or several (preferably one, two, three, or four) conservative substitutions.

An alkaline protease with a plurality of N-terminal amino acids and/or a plurality of C-terminal amino acids being deleted is also included in the alkaline protease of the present invention, as long as the effect of the present invention is achieved. An "amino acid sequence in which one or several amino acids are deleted from, substituted for, inserted into, and/or added to" or "amino acid sequence having 90% or more identity to" the amino acid sequence of the amino acid sequence set forth in SEQ ID NO: 1 is an amino acid sequence set forth in SEQ ID NO: 1 with a substitution, and the substitution in the amino acid sequence is a conservative substitution that retains a function of the alkaline protease. In other words, the phrase "conservative substitution" means a substitution that does not result in loss of an excellent effect of the alkaline protease. That is, it is a substitution that can recognize the amino acid sequence SLXX and cleaves between the two Xs even when an amino acid is deleted, substituted, or inserted, or added as described above. Specifically, it means replacing an amino acid residue with another chemically similar amino acid residue. For example, examples thereof include a case in which one hydrophobic residue is substituted with another hydrophobic residue, or a case in which one polar residue is substituted with another polar residue having the same charge. Functionally similar amino acids that can be substituted in such a way are known in the art for each amino acid. Examples of a non-polar (hydrophobic) amino acid include, for example, alanine, valine, isoleucine, leucine, proline, tryptophan, phenylalanine, methionine, etc. Examples of a polar (neutral) amino acid include, for example, glycine, serine, threonine, tyrosine, glutamine, asparagine, cysteine, etc. Examples of a positively charged (basic) amino acid include arginine, histidine, lysine, etc. Examples of a negatively charged (acidic) amino acid include aspartic acid, glutamic acid, etc.

The alkaline protease is not limited and is derived from koji mold. Specific examples thereof include Aspergillus flavus, Aspergillus clavatus, Aspergillus nidulans, Aspergillus funigatus, Aspergillus fischeri, or Aspergillus oryzae, with Aspergillus oryzae being preferred.

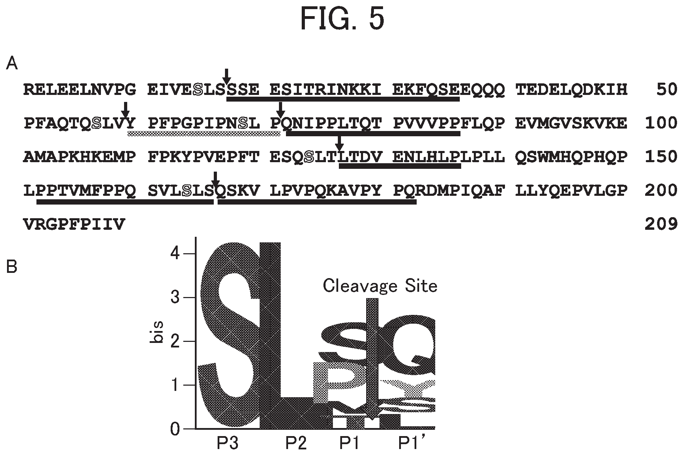

An alkaline protease to be used in the present invention recognizes SLXX and cleaves a peptide (protein) between XX. FIG. 5 shows cleavage sites for A2-type casein. That is, as shown in FIG. 5, the alkaline protease can recognize "SLSS" and cleave between SS, can recognize "SLVY" and cleave between VY, can recognize "SLPQ" and cleave between PQ, can recognize "SLTL" and cleave between TL, and can recognize "SLSQ" and cleave between SQ. Such an ability to recognize SLXX and cleave a peptide (protein) between XX allows production of an opioid peptide of the present invention from A2-type casein, as described below.

### <<A2-type casein>>

A2-type casein is A2-type β-casein included in milk. The β-casein is a protein composed of 224 amino acids and accounts for about 30% of the protein content of milk. There are two types of β-casein: A1-type casein (SEQ ID NO: 4) and A2-type casein (SEQ ID NO: 3). The A1-type casein and the A2-type casein differ in whether the 67th amino acid is histidine or proline. Thus, an opioid peptide set forth in SEQ ID NO: 2 is produced from the A2-type casein by means of the alkaline protease, but the opioid peptide is not produced from the A1-type casein. The A1-type casein is common in Holstein cattle, while the A2-type casein is common in Guernsey cattle. Therefore, milk containing only the A1-type casein, milk containing only the A2-type casein, or milk containing both the A1-type casein and the A2-type casein are commercially available.

### <<Opioid peptide>>

An opioid peptide obtained by the method for producing an opioid peptide of the present invention is not particularly limited as long as the effects of the present invention are achieved, but is preferably a peptide including an amino acid sequence set forth in SEQ ID NO: 2, that is, a peptide including an amino acid sequence "YPFPGPIPNSLP (hereinafter may be referred to as CM-12)". The opioid peptide is a peptide that has a binding activity to an opioid receptor. The opioid receptor is a cell surface receptor protein involved in exertion of an effect of a morphine-like substance and is a Gᵢ/Gₒ-coupled, 7-transmembrane receptor. Three types of opioid receptors: δ, κ, and µ receptors have been reported. An opioid receptor to which the opioid peptide of the present invention binds is not limited, but is preferably a δ receptor or a µ receptor. The δ receptor has a strong affinity for leucine-enkephalin and is widely distributed in the central nervous system. The δ receptor is also involved in an antidepressant effect, physical and mental dependence, and analgesia, and includes two receptors, δ₁ and δ₂. The opioid peptide of the present invention has a binding activity to an opioid receptor, especially the µ receptor and the δ receptor, and is expected to exert an anxiolytic, analgesic, antidepressant, or antistress effect through binding to the receptor.

### <<Peptide production step>>

In a step of producing an opioid peptide in the method for producing an opioid peptide of the present invention, a koji mold alkaline protease is brought into contact with A2-type casein to produce an opioid peptide including an amino acid sequence set forth in SEQ ID NO: 2. A condition under which the koji mold alkaline protease is brought into contact with the A2-type casein is not particularly limited, as long as the opioid peptide is produced. However, the pH of a buffer solution is, for example, pH 4 to 10, preferably 5 to 9.5, more preferably pH 6 to 9, further preferably pH 6.5 to 8.5, and most preferably pH 7 to 8. Specific examples of the buffer solution include an acetate buffer solution, a citrate buffer solution, or a tartrate buffer solution. A reaction temperature of the koji mold alkaline protease is also not particularly limited as long as the opioid peptide is produced and is, for example, 5 to 60°C, preferably 10 to 50°C, more preferably 20 to 48°C, more preferably 30 to 46°C, and further more preferably 35 to 42°C. A reaction time is also not particularly limited, and is, for example, a lower limit thereof is 10 minutes or more, in one aspect 30 minutes or more, and in one aspect 1 hour or more. An upper limit of the reaction time is, for example, 72 hours or less, in one aspect 48 hours or less, in one aspect 24 hours or less, and in one aspect 12 hours or less. Those skilled in the art can appropriately set the reaction pH, the reaction temperature, and the reaction time depending on koji mold alkaline proteases to be used.

### <<Method for improving production efficiency of opioid peptide>>

A method for improving production efficiency of an opioid peptide of the present invention includes bringing a koji mold alkaline protease that recognizes an amino acid sequence SLXX and cleaves between the two Xs into contact with A2-type casein, thereby producing an opioid peptide including an amino acid sequence set forth in SEQ ID NO: 2. In the method for improving production efficiency of an opioid peptide of the present invention, the koji mold alkaline protease is recovered as a low-adsorption fraction on an anion exchange resin or as an adsorption fraction on a cation exchange resin in order to separate the koji mold alkaline protease from a contaminant protease, although it is not limited thereto.

### <<Method for purifying (producing) alkaline protease>>

The koji mold alkaline protease can be purified (produced) from a mixture of koji mold proteases, although it is not limited thereto. The koji mold alkaline protease of the present invention has an alkaline isoelectric point. On the other hand, many koji mold proteases are acidic enzymes, and thus the koji mold alkaline protease of the present invention can be separated from these acidic proteases by an anion exchange resin or a cation exchange resin. For example, the anion exchange resin is difficult to adsorb the koji mold protease at a low pH. Specifically, the koji mold protease is adsorbed on the anion exchange resin at pH 8.5, but is eluted with 10 mM NaCl. It is eluted into a low adsorption fraction at a further lower pH. For example, when the koji mold protease is recovered as the low adsorption component on the anion adsorption resin, a buffer solution with a pH of 7 or more, preferably pH 8 or more may be used. In addition, when a cation exchange resin is used, the alkaline protease of the present invention can be adsorbed on the cation exchange resin. On the other hand, a koji mold acidic protease cannot be adsorbed on the cation exchange resin, and thus the alkaline protease can be separated from the acidic protease with the cation exchange resin. For example, when using as an adsorption fraction on a cation exchange resin, a buffer solution with pH 7 or less, preferably pH 6 or less may be used.

### [2] Alkaline protease composition for producing opioid peptide from A2-type casein

An alkaline protease composition of the present invention is used for producing an opioid peptide including an amino acid sequence set forth in SEQ ID NO: 2 from A2-type casein, the composition including a koji mold alkaline protease that recognizes an amino acid sequence SLXX and cleaves between the two Xs. The "A2-type casein" and the "opioid peptide" are the A2-type casein and the opioid peptide described in the above section "[1] Method for producing opioid peptide".

### <<Alkaline protease>>

An alkaline protease included in a composition of the present invention is a koji mold alkaline protease that recognizes an amino acid sequence SLXX and cleaves between the two Xs and the alkaline protease described in the section "[1] Method for producing opioid peptide" may be used without any limitation.

The alkaline protease can be obtained from a crudely purified enzyme derived from koji mold, although it is not limited thereto. The koji mold is preferably Aspergillus spp. and particularly preferably Aspergillus oryzae. For example, the alkaline protease can be efficiently purified from a crudely purified enzyme derived from Aspergillus oryzae by means of DEAE-Sepharose chromatography and elution with 10 mM NaCl. At a lower pH, the alkaline protease is eluted into a low adsorption fraction, and productivity of the opioid peptide of the present invention can be enhanced by removing other contaminant proteases.

In addition to the alkaline protease, the composition of the present invention may include a carrier or other components, etc. For example, a water-soluble solvent such as saline or Ringer's solution, a water-insoluble solvent such as a vegetable oil or a fatty acid ester, an isotonic agent such as glucose or sodium chloride, a dissolution aid, a stabilizing agent, a preservative, a suspending agent, or an emulsifying agent, etc., can optionally be used as the other components. A content of the alkaline phosphatase in the composition of the present invention is not particularly limited, and the alkaline protease can be included at a concentration of 90% by weight or more, 50% by weight or more, 10% by weight or more, or 1% by weight or more, with an upper limit thereof being less than 100% by weight or less than 99% by weight.

### [3] Opioid peptide

An opioid peptide of the present invention is an opioid peptide having an amino acid sequence set forth in SEQ ID NO: 2. The opioid peptide described in the above section "[1] Method for producing opioid peptide" can be used as the opioid peptide without any limitation. The opioid peptide can be synthesized, but is preferably produced by a method for producing an opioid peptide of the present invention.

### [4] Opioid receptor agonistic agent

An opioid receptor agonistic agent of the present invention includes an opioid peptide of the present invention as an active ingredient. In other words, the opioid receptor agonistic agent of the present invention includes an opioid peptide having an amino acid sequence set forth in SEQ ID NO: 2 as an active ingredient. The opioid receptor agonistic agent of the present invention is an agonist and can exhibit an analgesic, antidepressant, or antistress effect by binding to an opioid receptor. The opioid receptor includes a δ receptor, a κ receptor, and a µ receptor. An opioid receptor on which the peptide of the present invention acts is not limited, but is preferably a δ receptor or a µ receptor.

### [5] Food composition

A food composition for agonizing an opioid receptor or an anti-anxiety food composition includes an opioid peptide of the present invention as an active ingredient.

Specific examples of food include fresh food such as salad; cooked food such as steak, pizza, or hamburger; stir-fried food such as stir-fried vegetable; a vegetable such as a tomato, a pepper, a celery, a bitter gourd, a carrot, a potato, or an asparagus, or cooked food processed from these vegetables; confectionery such as cookie, bread, biscuit, hardtack, cake, rice cracker, yokan jelly, pudding, jelly, ice cream, chewing gum, cracker, chips, chocolate, or candy; noodle such as udon, pasta, or soba; fish cake such as fish paste, fish ham, or fish sausage; dairy products such as cheese, cream, or butter; seasonings such as miso, soy sauce, dressing, ketchup, mayonnaise, soup stock, seasoning soy sauce, curry powder, sweet cooking rice wine, roux, or seasoning spice; soy food such as tofu; konjac; a dietary supplement, etc.

Examples of a beverage may include, for example, coffee drink; cocoa drink; vegetable juices processed from the above-mentioned vegetables; fruit juice such as grapefruit juice, orange juice, grape juice, or lemon juice; tea drink such as green tea, black tea, sencha tea, or oolong tea; an alcoholic beverage such as beer, wine (red wine, white wine, or sparkling wine), sake, plum wine, low-malt beer, whiskey, brandy, shochu, rum, gin, or liqueur; dairy drink; soy milk drink; liquid diet; a sports beverage, etc.

The food or beverage includes feed for an animal. An animal of interest includes, for example, a primate such as a human, cattle, a pig, a sheep, a goat, a horse, a dog, a cat, a rabbit, a rat, a mouse, etc.

The food or beverage may contain, if desired, a food additive and a food material such as an antioxidant, a flavoring agent, an acidulant, a coloring agent, an emulsifying agent, a preservative, a seasoning, a sweetener, a spice, a pH adjusting agent, a stabilizing agent, an antioxidant, a vegetable oil, an animal oil, a sugar and a sugar alcohol, a vitamin, an organic acid, a fruit juice extract, a vegetable extract, a grain, a bean, a vegetable, meat, fish and seafood alone or in combination of two or more thereof. An amount of the food material or the food additive to be incorporated can be determined as appropriate, unless the object of the present invention is not impaired.

The food or beverage includes functional food (beverage) and health food (beverage). As used herein, the phrase "health food (beverage)" means food or beverage that provides or can be expected to provide some effect on health, and the phrase "functional food (beverage)" means, among the "health food (beverage)", food or beverage designed and processed to fully exert a bioregulatory function (i.e., opioid receptor agonistic effect). The functional food and the health food can be in a granular, solid, liquid, capsule, gel, or tablet form.

### [6] Pharmaceutical composition

A pharmaceutical composition for agonizing an opioid receptor or an anti-anxiety pharmaceutical composition of the present invention includes an opioid peptide of the present invention as an active ingredient. The pharmaceutical composition for agonizing an opioid receptor of the present invention can be used for, but is not limited to, an anxiolytic, analgesic, antidepressant, or anti-stress application.

A dosage form of the pharmaceutical composition of the present invention is not particularly limited, and examples thereof include an oral preparation or a parenteral preparation, with the oral preparation being preferred. The oral preparation includes, for example, a solid or powder preparation such as a fine granule, a granule, a tablet, a capsule, or a pill, as well as a liquid preparation such as a suspension, an emulsion, a syrup, or an extract. The parenteral preparation includes, for example, an injection.

The pharmaceutical composition of the present invention may consist of or include the opioid peptide. When the pharmaceutical composition of the present invention includes the opioid peptide, another additive may be included.

When the pharmaceutical composition of the present invention is an oral preparation, the other additive may include an excipient, a binder, a disintegrant, an emulsifying agent, a lubricant, a glidant, a diluent, a preservative, a colorant, a flavoring agent, a corrigent, a stabilizing agent, a moisturizer, an antiseptic, an antioxidant, or a suspending agent, specifically, gelatin, sodium alginate, starch, cornstarch, white soft sugar, lactose, glucose, mannitol, carboxymethyl cellulose, dextrin, polyvinylpyrrolidone, crystalline cellulose, soy lecithin, sucrose, a fatty acid ester, talc, magnesium stearate, polyethylene glycol, magnesium silicate, silicic anhydride, or synthetic aluminum silicate.

When the pharmaceutical composition of the present invention is a parenteral preparation, the other additive may include a water-soluble solvent such as saline or Ringer's solution, a water-insoluble solvent such as a vegetable oil or a fatty acid ester, an isotonic agent such as glucose or sodium chloride, a dissolution aid, a stabilizing agent, a preservative, a suspending agent, or an emulsifying agent, etc.

The pharmaceutical compositions of the present invention may include 90% by weight or more, 50% by weight or more, 10% by weight or more, or 1% by weight or more of the peptide or a mixture of the peptide.

A dosage or intake of the pharmaceutical composition of the present invention can be appropriately adjusted depending on a form of preparation, as well as an age, a sex, a weight, or a degree of a symptom of a disease of a subject for which the composition is used, but is preferably an amount that can exhibit an analgesic, antidepressant, or anti-stress effect by administration or intake of the pharmaceutical composition. Specifically, in terms of an amount of the opioid peptide to be added, it may be 0.01 to 1000 mg/kg body weight/day, preferably 0.1 to 750 mg/kg body weight/day, more preferably 1 to 500 mg/kg body weight/day, further preferably 5 to 400 mg/kg body weight/day, further preferably 10 to 300 mg/kg body weight/day, further preferably 15 to 200 mg/kg body weight/day, or most preferably 20 to 150 mg/kg body weight/day.

Of course, the above-mentioned administration method is merely an example and other administration methods may also be used. An administration method, a dosage, a dosing period, or an interval of administration of the pharmaceutical composition to a human is desirably determined by a controlled clinical trial.

The pharmaceutical composition of the present invention can be administered to a human, and may also be administered to an animal other than a human including a pet such as a dog, a cat, a rabbit, a hamster, a guinea pig, or a squirrel; a livestock such as cattle or a pig; a laboratory animal such as a mouse or a rat; or an animal kept in a zoo, etc.

The pharmaceutical composition includes a pharmaceutical and a quasi-drug. The pharmaceutical includes, for example, a crude drug preparation or a Chinese herbal drug preparation. The quasi-drug includes, for example, an energy drink or a crude drug-containing supplement.

### EXAMPLES

Hereinafter, the present invention will be specifically described with reference to Examples, but the present invention is not limited thereto.

### <<Example 1>>

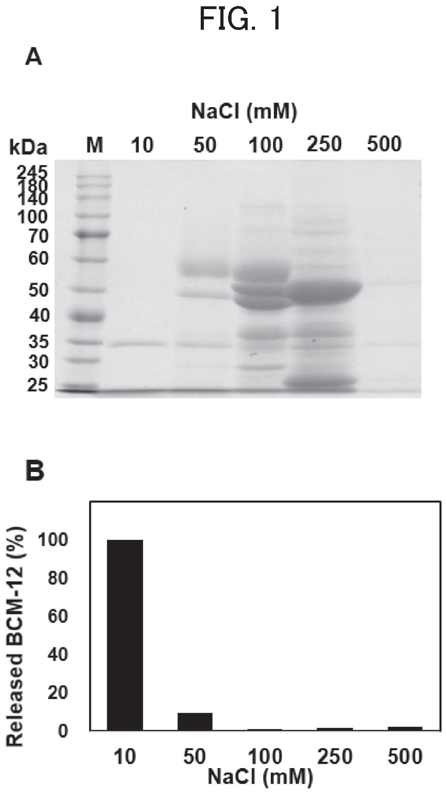

In this example, an alkaline protease to be used in the present invention was purified from a crudely purified enzyme derived from Aspergillus oryzae. First, 50 mg of the crudely purified enzyme was dissolved in 2 mL of a 100 mM Tris-HCl buffer solution (pH 8.5, 20°C). The resulting crudely purified enzyme solution was applied to DEAE-Sepharose Fast Flow anion exchange chromatography (1 mL) pre-equilibrated with a 100 mM Tris-HCl buffer solution (10 mL). A protein was eluted from a column stepwise with 10, 50, 100, 250, and 500 mM NaCl in 100 mM Tris-HCl. A protein concentration was estimated by measuring absorbance at 280 nm. The resulting fractions were analyzed by SDS-PAGE. A photograph of a gel was obtained as shown in FIG. 1(A).

Each fraction was brought into contact with an amino acid sequence AQTQSLVYPFPGPIPNSLPQNIPPLTQTPV (CM-30) (SEQ ID NO: 5) prepared from an A2-type β-casein sequence to examine an ability to produce an opioid peptide of the present invention. As shown in FIG. 1(B), an activity to produce CM-12 (YPFPGPIPNSLP) from the CM-30 was confirmed in a 10 mM NaCl fraction. In the following examples, this fraction was used as an alkaline protease.

A band of a 34 kDa protein in the 10 mM NaCl fraction was isolated from an SDS-10% PAGE gel. A peptide obtained from the protein by trypsin digestion was analyzed by TOF-MS analysis. Mass data of the resulting tryptic peptides were analyzed by the MASCOT database. The 34 kDa protein was believed to be the alkaline protease set forth in SEQ ID NO: 1. That is, it was believed to be a 283 aa mature protease excluding a 21 aa signal sequence and a pro-sequence that is 100 aa from the N-terminus.

### <<Example 2>>

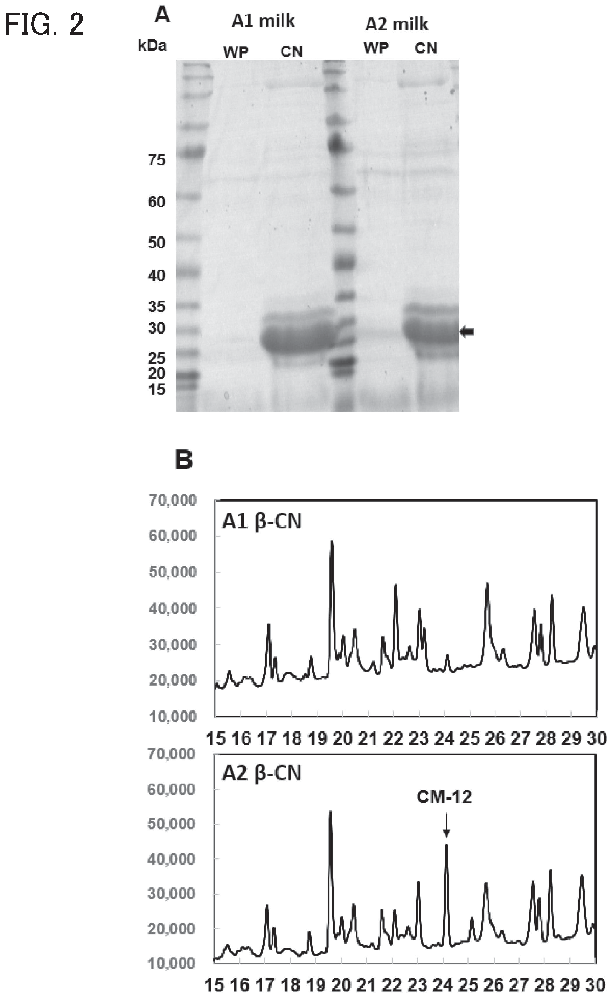

In this example, cleavage of an opioid peptide of the present invention from A1-type casein or A2-type casein was examined using the alkaline protease obtained in Example 1. The A1-type casein was used as a comparative example. Ten milliliters of A1-type milk or A2-type milk was centrifuged at 7,500 x g, 4°C for 20 minutes to remove a cream top layer. Each supernatant was heated at 55°C for 5 minutes and cooled to 20°C or less. The pH was then adjusted to 4.6 by adding 1 M hydrochloric acid to the supernatant, and aggregates were collected by centrifugation at 1,300 x g, 20°C for 5 minutes. To dissolve a pellet, a precipitate was dissolved in 10 mL of distilled water, and the pH was adjusted to 7.0 by adding 1 M NaOH thereto. The resulting casein protein was analyzed by SDS-12% PAGE (FIG. 2A). Bands (arrows) corresponding to the A1-type casein and the A2-type casein were observed. The A1-type casein and the A2-type casein were isolated, and a CM-12 release capacity of purified Alp was evaluated. As a result, a specific peak corresponding to the CM-12 was detected in an A2 casein hydrolysate (FIG. 2B), but not in an A1 casein hydrolysate (FIG. 2B).

### <<Example 3>>

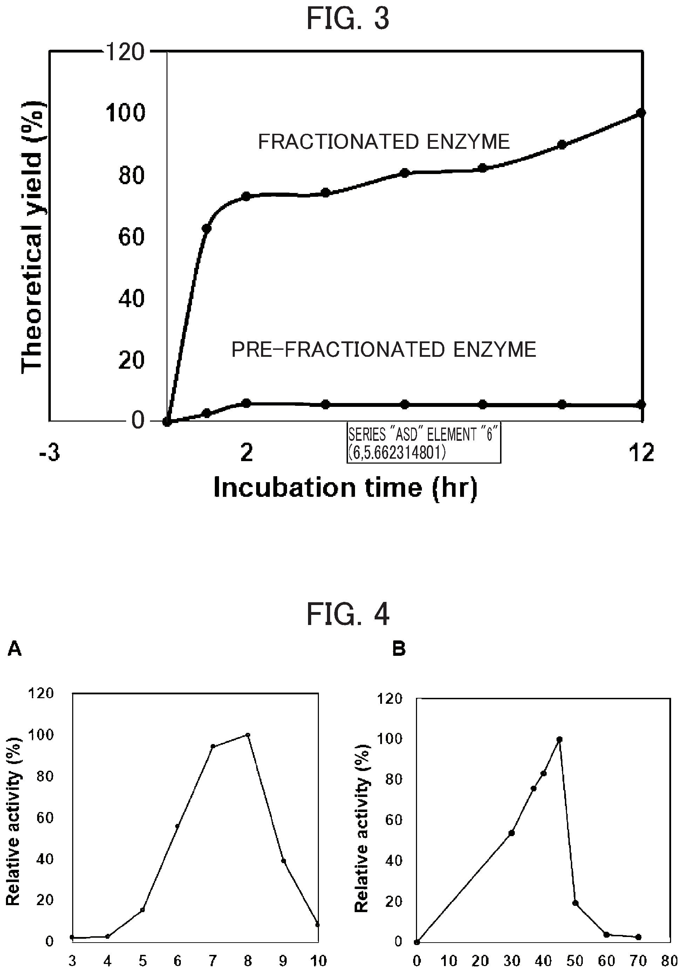

In this example, production of a CM-12 peptide from A2-type casein using a purified alkaline protease and a crudely purified enzyme was examined. Three microliters of the A2-type casein (2 mg/mL) was hydrolyzed with 10 µL of a purified alkaline protease (0.3 mg/mL) or 10 µL of a crudely purified enzyme (30 mg/mL), and the pH was adjusted to 8 with a 0.2 M Na₂HPO₄ buffer. The resulting reaction mixture was incubated at 45°C for 1, 2, 4, 6, 8, 10, and 12 hours, and the opioid peptide was measured. The crudely purified enzyme produced a very low level of CM-12, but the purified alkaline protease produced CM-12 for a long time. The purified alkaline protease was able to release 100%, which corresponded to 18.6-fold, of CM-12 from the A2-type casein (FIG. 3).

### <<Example 4>>

In this example, a reaction pH and a reaction temperature of the alkaline protease obtained in Example 1 were examined. Specifically, CM-30 was used as a substrate and an activity of the purified alkaline protease was examined. For pH 3.0 to 5.0, a 0.2 M citric acid-Na₂HPO₄ buffer solution was used; for pH 6.0 to 8.0, a 0.2 M Na₂HPO₄-NaH₂PO₄ buffer solution was used; and for pH 9.0 and 10.0, a glycine-sodium hydroxide buffer solution was used. The purified alkaline protease was incubated with a substrate at a 10:1 (S/E, w/w) ratio in a reaction buffer at 45°C for 1 hour. As shown in FIG. 4A, an optimum pH was around 8. For the reaction temperature, 0.2 M Na₂HPO₄-NaH₂PO₄ buffer solution, pH 8.0 was used, and the activity was tested at 30, 37, 40, 45, 50, 60, and 70°C. The alkaline protease was incubated at a ratio of 10:1 (S/E, w/w) for 1 hour. As shown in FIG. 4B, an optimum temperature was around 45°C.

### INDUSTRIAL APPLICABILITY

A method for producing an opioid peptide of the present invention is effective for production of an opioid peptide for use in foods or medicaments for applications such as anxiolytic, analgesic, antidepressant, or anti-stress applications.

## Claims

1. A method for producing an opioid peptide, the method comprising
bringing a koji mold alkaline protease that recognizes an amino acid sequence SLXX and cleaves between the two Xs into contact with A2-type casein, thereby producing a peptide comprising an amino acid sequence set forth in SEQ ID NO: 2.

2. The method for producing an opioid peptide according to claim 1, wherein the koji mold alkaline protease is (a) a koji mold alkaline protease having an amino acid sequence set forth in SEQ ID NO: 1, or (b) a koji mold alkaline protease comprising an amino acid sequence having 90% or more identity to the amino acid sequence set forth in SEQ ID NO: 1 and capable of producing from the A2-type casein an opioid peptide comprising the amino acid sequence set forth in SEQ ID NO: 2.

3. A method for improving production efficiency of an opioid peptide, comprising
bringing a koji mold alkaline protease that recognizes an amino acid sequence SLXX and cleaves between the two Xs into contact with A2-type casein, thereby producing an opioid peptide comprising an amino acid sequence set forth in SEQ ID NO: 2,
the koji mold alkaline protease being recovered as a low-adsorption fraction on an anion exchange resin or as an adsorption fraction on a cation exchange resin in order to separate the koji mold alkaline protease from a contaminant protease.

4. The method for improving production efficiency of an opioid peptide according to claim 3, wherein the koji mold alkaline protease is (a) a koji mold alkaline protease having an amino acid sequence set forth in SEQ ID NO: 1, or (b) a koji mold alkaline protease comprising an amino acid sequence having 90% or more identity to the amino acid sequence set forth in SEQ ID NO: 1 and capable of producing from the A2-type casein an opioid peptide comprising the amino acid sequence set forth in SEQ ID NO: 2.

5. An alkaline protease composition for producing an opioid peptide comprising an amino acid sequence set forth in SEQ ID NO: 2 from A2-type casein, the composition comprising a koji mold alkaline protease that recognizes an amino acid sequence SLXX and cleaves between the two Xs.

6. The alkaline protease composition according to claim 5, wherein the koji mold alkaline protease is (a) a koji mold alkaline protease having an amino acid sequence set forth in SEQ ID NO: 1, or (b) a koji mold alkaline protease comprising an amino acid sequence having 90% or more identity to the amino acid sequence set forth in SEQ ID NO: 1 and capable of producing from the A2-type casein an opioid peptide comprising the amino acid sequence set forth in SEQ ID NO: 2.

7. An opioid peptide having an amino acid sequence set forth in SEQ ID NO: 2.

8. An opioid receptor agonistic agent comprising as an active ingredient the opioid peptide according to claim 7.

9. A food composition for agonizing an opioid receptor, the composition comprising as an active ingredient the opioid peptide according to claim 7.

10. An anti-anxiety food composition comprising as an active ingredient the opioid peptide according to claim 7.

11. A pharmaceutical composition for agonizing an opioid receptor, the composition comprising as an active ingredient the opioid peptide according to claim 7.

12. An anti-anxiety pharmaceutical composition including as an active ingredient the opioid peptide according to claim 7.
